(19) Europäisches Patentamt
European Patent Office
Office européen des brevets

(11) EP 4 070 823 B1

(12) **EUROPÄISCHE PATENTSCHRIFT**

(45) Veröffentlichungstag und Bekanntmachung des Hinweises auf die Patenterteilung:
**21.02.2024 Patentblatt 2024/08**

(21) Anmeldenummer: **22164499.0**

(22) Anmeldetag: **25.03.2022**

(51) Internationale Patentklassifikation (IPC):
***A61L 27/34*** *(2006.01)* ***A61L 27/54*** *(2006.01)*

(52) Gemeinsame Patentklassifikation (CPC):
(C-Sets verfügbar)
**A61L 27/34; A61L 27/54;** A61L 2300/204;
A61L 2300/404 (Forts.)

(54) **ANTIBAKTERIELLE IMPLANTATBESCHICHTUNGSZUSAMMENSETZUNG, VERFAHREN ZUM BESCHICHTEN EINES IMPLANTATS UND ANTIBAKTERIELL BESCHICHTETES IMPLANTAT**

ANTIBACTERIAL IMPLANT COATING COMPOSITION, METHOD OF COATING AN IMPLANT AND ANTIBACTERIAL COATED IMPLANT

COMPOSITION ANTIBACTÉRIENNE DE REVÊTEMENT D'IMPLANT, PROCÉDÉ DE REVÊTEMENT D'UN IMPLANT ET IMPLANT REVÊTU DE MANIÈRE ANTIBACTÉRIENNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.04.2021 DE 102021108936**

(43) Veröffentlichungstag der Anmeldung:
**12.10.2022 Patentblatt 2022/41**

(73) Patentinhaber: **Gebr. Brasseler GmbH & Co. KG**
**32657 Lemgo (DE)**

(72) Erfinder:
- **SEIFFERT, Anne**
**38116 Braunschweig (DE)**
- **MENZEL, Henning**
**31275 Lehrte (DE)**
- **KÜLLMER, Michael**
**32657 Lemgo (DE)**
- **SCHWARZ, Hans-Christoph**
**32107 Bad Salzuflen (DE)**
- **STIESCH, Meike**
**30177 Hannover (DE)**
- **WINKEL, Andreas**
**30163 Hannover (DE)**
- **GRISCHKE, Jasmin**
**30657 Hannover (DE)**

(74) Vertreter: **Hoefer & Partner Patentanwälte mbB**
**Pilgersheimer Straße 20**
**81543 München (DE)**

(56) Entgegenhaltungen:
**EP-A1- 3 153 186**

EP 4 070 823 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)

C-Sets
**A61L 27/34, C08L 33/02**

## Beschreibung

Die Erfindung betrifft eine antibakterielle Implantatbeschichtungszusammensetzung mit sehr guter antibakterieller Wirkung und Haftung an einem Implantat. Darüber hinaus betrifft die vorliegende Erfindung auch ein Verfahren zum Beschichten eines Implantats sowie ein antibakteriell beschichtetes Implantat.

Implantate werden in vielen medizinischen Disziplinen zur Rekonstruktion von Organ- und Gewebefunktionen eingesetzt. Sie bestehen aus artifiziellen Materialien, an denen Bakterien anhaften und sich in komplexen Biofilmgemeinschaften organisieren können. Die in der Folge entstehenden Entzündungsreaktionen und damit verbundenen fortschreitenden destruktiven Prozesse im umgebenden Gewebe führen zu Funktionsverlusten des Implantates und erheblichen Beeinträchtigungen des Patienten. Insbesondere in der Zahnmedizin sind periimplantäre Infektionen mit einer Prävalenz von 30 % von großer klinischer Bedeutung. Aufgrund der hohen Resistenz bakterieller Biofilme gegenüber chemischen Therapeutika erfolgt die Biofilmentfernung in der Zahnmedizin vorwiegend mechanisch, wobei viele Bereiche des zahnärztlichen Implantates aufgrund der komplexen Geometrie nur unvollständig erreichbar sind.

Das Problem der implantatassoziierten Infektionen wird zurzeit durch eine Beschichtung des Implantats mit Silber bekämpft. Die Silberschicht verhindert eine Bakterienadhäsion, allerdings auch die Adhäsion von körpereigenen Zellen, und ist somit nicht selektiv. Eine weitere Neuentwicklung sind antibakterielle Polymere, die auf die Implantatmaterialien aufgebracht werden. Bisher entwickelte Polymere weisen aber entweder keine ausreichende Zellkompatibilität oder keine hinreichende antibakterielle Wirkung auf. Ein weiteres Problem ist die sichere und technisch umsetzbare Applikation der Polymere auf der Oberfläche.

Ausgehend von diesem Stand der Technik ist es eine Aufgabe der vorliegenden Erfindung eine antibakterielle Implantatbeschichtungszusammensetzung anzugeben, die einfach applizierbar ist, auf dem Implantat gut haftet und bei guter antibakterieller Wirkung sehr gut zellkompatibel ist. Eine weitere Aufgabe der vorliegenden Erfindung ist es, ein Verfahren zum Beschichten eines Implantats mit einer antibakteriellen Beschichtung anzugeben, das eine gleichmäßig abdeckende, am Implantat gut anhaftende Beschichtung erzeugt und dabei einen hohen technischen Aufwand bei einfacher Umsetzung vermeidet. Darüber hinaus ist es eine Aufgabe der vorliegenden Erfindung ein antibakteriell beschichtetes Implantat anzugeben, das sich bei hoher Zellkompatibilität durch eine sehr gute antibakterielle Funktionalität auszeichnet.

Die Lösung dieser Aufgaben erfolgt durch die Merkmale der unabhängigen Ansprüche. Die abhängigen Ansprüche haben vorteilhafte Weiterbildungen zum Inhalt.

Demnach wird die Aufgabe durch eine antibakterielle Implantatbeschichtungszusammensetzung gelöst, die ein spezifisches Pfropfcopolymer umfasst. Das Pfropfcopolymer weist dabei ein auf (Meth)Acrylsäure basierendes Rückgrat auf.

Unter allgemein "(Meth)Acrylsäure basierendes Rückgrat" und dergleichen, wird gemäß der vorliegenden Erfindung sowohl eine auf Propensäure, also Acrylsäure, basierte Verbindung als auch eine auf 2-Methylpropensäure, also Methacrylsäure, basierte Verbindung verstanden, nämlich gemäß dem o.g. Beispiel: "Acrylsäure basiertes Rückgrat" und "Methacrylsäure basiertes Rückgrat". Der Einfachheit halber wird in der nachfolgenden Beschreibung die Abkürzung "(Meth)Acrylsäure ..." in Vertretung für "Acrylsäure ... und "Methacrylsäure ..." verwendet.

An dem (Meth)Acrylsäure basierten Rückgrat sind Phosphonat-Seitenketten angeordnet. Mit anderen Worten liegt in dem Pfropfcopolymer ein Poly(meth)acrylsäure-Rückgrat vor, wobei die Phosphonat-Seitenketten mit den Säuregruppen bzw. Estergruppen des Poly(meth)acrylsäure-Rückgrats verbunden sind. Hierbei müssen die Phosphonat-Seitenketten nicht direkt mit den Säuregruppen bzw. Estergruppen abreagiert sein, sondern es kann zwischen dem Phosphoratom der Phosphonat-Seitenketten und dem einfachgebundenen Sauerstoff der Carboxylgruppe bzw. Estergruppe des Poly(meth)acrylsäure-Rückgrats auch eine divalente Verbindungsgruppe, wie insbesondere eine Alkylengruppe, die insbesondere 1 bis 8 Kohlenstoffatome und vorzugsweise 1 bis 4 Kohlenstoffatome umfasst, vorliegen. Die divalente Verbindungsgruppe hat jedoch keinen Einfluss auf die antibakterielle Funktionalität des Pfropfcopolymers.

Vielmehr geht die antibakterielle Funktionalität des Pfropfcopolymers von einem mit der Phosphonat-Seitenkette verbundenen Guanidin-Oligomer aus. Unter Guanidin-Oligomer werden im Sinne der vorliegenden Erfindung Derivate von Oligoguanidinen verstanden, die insbesondere durch Reaktion von Diaminen mit Guanidinhydrochlorid synthetisierbar sind. Oligoguanidine und Polyguanidine sind zur Bekämpfung von Mikroorganismen bekannt und werden oftmals in Desinfektionsmitteln, Wasch- und Reinigungsmitteln oder Kosmetika eingesetzt. Aufgrund ihrer hohen Wasserlöslichkeit eignen sie sich in der bisher angewendeten Form nicht zur Beschichtung von Implantaten.

In dem erfindungsgemäß verwendeten Pfropfcopolymer ist mindestens eine Phosphonat-Seitenkette mit einem Guanidin-Oligomer durch eine N-P-Bindung verbunden. Bei der N-P-Bindung handelt es sich um eine kovalente Bindung zwischen einem Stickstoffatom des Guanidin-Oligomers und dem Phosphoratom einer Phosphonat-Seitenkette. Die hierbei entstehende Verbindungsgruppe ist insbesondere eine Phosphonamidatgruppe. Dies bedeutet, dass vorzugsweise eine primäre Amingruppe mit der Phosphonatgruppe umgesetzt ist, woraus sich die vorstehend beschriebene direkte und kovalente N-P-Bindung ergibt.

In dem erfindungsgemäß verwendeten Pfropfcopolymer kann eine Phosphonatgruppe oder können mehrere

Phosphonatgruppen mit einem oder mehreren Guanidin-Oligomeren umgesetzt und verbunden sein. Je höher der Anteil an Guanidin-Oligomer-Resten ist, desto höher ist die antibakterielle Schutzwirkung, die die antibakterielle Implantatbeschichtungszusammensetzung entfalten kann. Eine antibakterielle Wirkung wird jedoch auch schon bei sehr geringen Anteilen an Guanidin-Oligomer im Pfropfcopolymer erhalten und kann, wie dargelegt, durch einen höheren Anteil an N-P-verbundenen Guanidin-Oligomer-Gruppen erhöht werden.

In dem für die antibakterielle Implantatbeschichtungszusammensetzung verwendeten Pfropfcopolymer dient das auf (Meth)Acrylsäure basierende Rückgrat zum einen der Stabilität der Implantatbeschichtungszusammensetzung, und zum anderen der Bereitstellung von Phosphonat-Seitenketten in ausreichender Anzahl. Durch die Phosphonat-Seitenketten erhält das Pfropfcopolymer eine Ankerfunktion zum Binden an übliche Implantatmaterialien wie z.B. Titan, Edelstahl, Zirkon, Tantal, Zirkonoxid, PEEK und dergleichen. Die erfindungsgemäße Implantatbeschichtungszusammensetzung ist somit selbstbindend. Es bedarf keiner zusätzlichen adhäsiven Komponente. Dies erleichtert eine Applikation der Implantatbeschichtungszusammensetzung auf dem Implantat und ermöglicht Beschichtungen auch auf geometrisch komplexen Implantatoberflächen.

Die antibakterielle Wirkung der Implantatbeschichtungszusammensetzung wird durch das oder die kovalent gebundenen Guanidin-Oligomer-Reste erzielt, die aufgrund der kovalenten Bindung nicht mehr wasserlöslich sind und damit nach Applikation auf einem Implantat dauerhaft dort verweilen und ihre antibakterielle Wirkung entfalten.

Die erfindungsgemäße Implantatbeschichtungszusammensetzung zeichnet sich somit durch eine hohe Funktionalität aus und ist dabei doch einfach strukturiert, da sie im Wesentlichen als funktionelle Komponente das vorstehend beschriebene Pfropfcopolymer enthält. Die antibakterielle Implantatbeschichtungszusammensetzung ist damit einfach verarbeitbar und insbesondere gleichmäßig mit gewünschter Schichtdicke applizierbar, haftet auf dem Implantat gut und dauerhaft an und ist bei sehr guter antibakterieller Wirkung hoch zellkompatibel.

Zur Optimierung der antibakteriellen Eigenschaften der Implantatbeschichtungszusammensetzung bei gleichzeitig sehr guter Haftung auf einem Implantat, beträgt ein Anteil an Phosphonat-Seitenketten, die mit einem Guanidin-Oligomer durch eine (kovalent) N-P-Bindung verbunden sind, bezogen auf den Gesamtanteil an Phosphonat-Seitengruppen in Mol%, vorzugsweise 0,8 bis 6 Mol% und insbesondere 0,9 bis 5 Mol%. Bereits sehr geringe Anteile an Phosphonat-Seitenketten, die mit einem Guanidin-Oligomer kovalent verbunden sind, von 0,8 Mol% erzielen eine besonders gute und dauerhaft beständige antibakterielle Wirkung durch die hohe antibakterielle Funktionsfähigkeit von Guanidin-Oligomeren. Die restlichen Phosphonat-Seitenketten, die nicht mit einem Guanidin-Oligomer verbunden sind, können vorzugsweise der Bindung an das Implantat dienen. So wird eine gute Balance zwischen ausreichender Haftung an einem Implantat und sehr guter antibakterieller Wirksamkeit bei gleichzeitig hoher Zellkompatibilität erzielt. Ein wie vorstehend offenbarter Mol% Anteil von 0,8 bis 6 Mol% an Phosphonat-Seitenketten, die mit einem Guanidin-Oligomer durch eine N-P-Bindung verbunden sind, entspricht dabei in etwa einem Masseanteil von 4 bis 30 Masse%, bezogen auf die Gesamtmasse aller Phosphonat-Seitenketten in dem Pfropfcopolymer.

Gemäß einer weiteren vorteilhaften Weiterbildung sind die nicht mit dem Guanidin-Oligomer verbundenen Phosphonat-Seitenketten nicht hydrolysiert, teilhydrolysiert, so dass sie hemi-Phosphonsäurereste bilden, oder vollhydrolysiert, so dass sie Phosphonsäurereste bilden. Hierdurch wird die Funktionalität des Pfropfcopolymers im Wesentlichen nicht beeinflusst. Allenfalls kann durch einen höheren Hydrolysegrad der Phosphonat-Seitenketten die Wasserdispergierbarkeit des Pfropfcopolymers verbessert werden.

Vorzugsweise beträgt hierbei ein Anteil an teilhydrolysierten und vollhydrolysierten Phosphonat-Seitengruppen, bezogen auf den Gesamtanteil an Phosphonat-Seitengruppen in Mol%, 5 bis 30 Mol% und insbesondere 7 bis 20 Mol%. Hierdurch können sehr gute Dispergiereigenschaften in polaren Lösungsmitteln erzielt werden. Ein Mol% Anteil von 5 bis 30 Mol% an teilhydrolysierten und vollhydrolysierten Phosphonat-Seitengruppen, bezogen auf den Gesamtanteil an Phosphonat-Seitengruppen, entspricht dabei in etwa 6 bis 25 Masse%, bezogen auf die Gesamtmasse aller Phosphonat-Seitenketten im Pfropfcopolymer.

Weiter vorteilhaft weist das erfindungsgemäß verwendete Pfropfcopolymer ein Molekulargewicht (Mw) auf, das nachfolgende Gleichung erfüllt:

$$5kDa < Mw < 40kDa.$$

Das Molekulargewicht kann dabei aus DOSY-NMR-Daten rechnerisch ermittelt werden. Ein Molekulargewicht im angegebenen Bereich hat den Vorteil einerseits hoch genug zu sein, um einen gut haftenden Film der antibakteriellen Implantatbeschichtungszusammensetzung auf einem Implantat zu ermöglichen, und andererseits niedrig genug zu sein, um eine gute Verarbeitbarkeit, insbesondere eine hohe Dispergierfähigkeit in Lösungsmitteln bereitzustellen, so dass die antibakterielle Implantatbeschichtungszusammensetzung gleichmäßig mit gewünschter Schichtdicke applizierbar ist.

Vorzugsweise ist das Guanidin-Oligomer mindestens eine durch die nachfolgenden Formeln dargestellte Verbindung, da sich diese Guanidin-Oligomere durch eine sehr gute Verbindbarkeit mit den Phosphonat-Seitenketten und hohe antibakterielle Wirksamkeit auszeichnen. Hierbei kann in dem Pfropfcopolymer lediglich ein spezifisches Guanidin-Oligomer verwendet werden oder aber eine Kombination von zwei oder mehreren Guanidin-Oligomeren der nachstehenden Formeln:

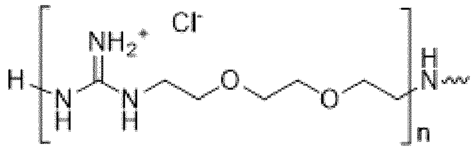

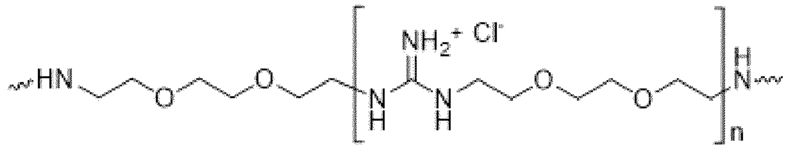

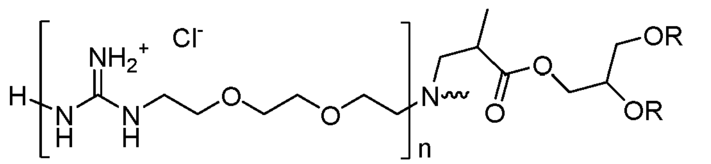

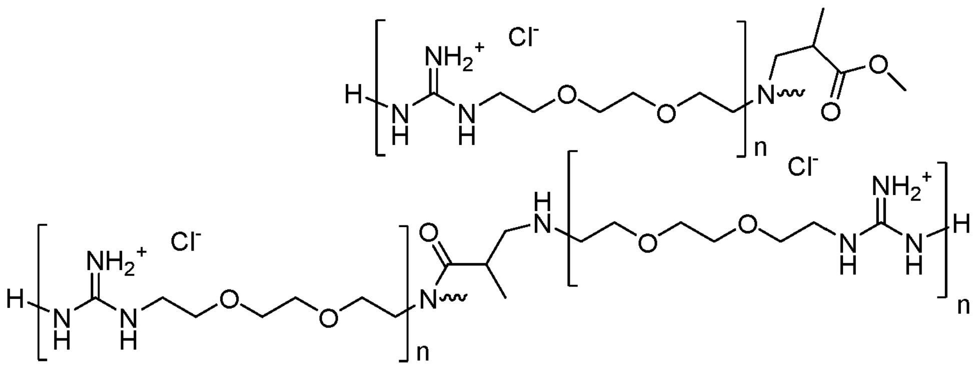

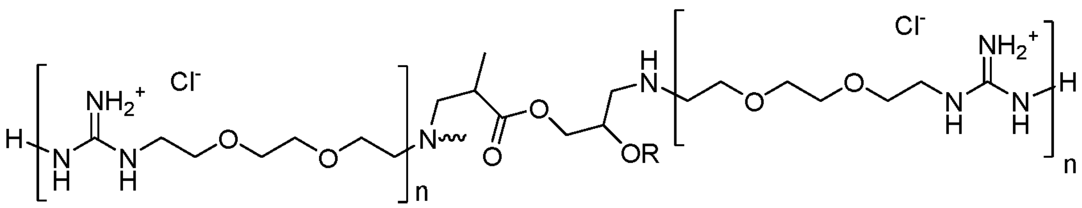

In den vorstehend offenbarten Formeln bezeichnet n jeweils eine ganze Zahl von 2 bis 10, vorzugsweise von 3 bis 6, und insbesondere 5, und eine Welle, "~", bezeichnet eine bevorzugte Bindungsposition des entsprechenden Stickstoffs an das Phosphoratom einer Phosphonat-Seitenkette. Wie in den oben dargestellten Formeln gezeigt, verfügen die Guanidin-Oligomere teilweise über mehrere primäre und auch sekundäre Amingruppen, die prinzipiell geeignet sind, mit einem Phosphoratom einer Phosphonat-Seitenkette zu reagieren. Vorzugsweise findet aber eine Reaktion an demjenigen Stickstoffatom statt, das mit einer Welle, "~", gekennzeichnet ist.

Ebenfalls im Lichte einer sehr guten Dispergierbarkeit des Pfropfcopolymers, beträgt das Molekulargewicht des Guanidin-Oligomers vorzugsweise 800 bis 1300 g/mol. Bei der Berechnung des Molekulargewichts des Guanidin-Oligomers wird der Guanidin-Oligomer-Rest betrachtet, wie er an dem Phosphoratom der Phosphonat-Seitenkette gebunden ist.

Ferner vorteilhaft weist das Pfropfcopolymer nachfolgende Struktureinheit auf:

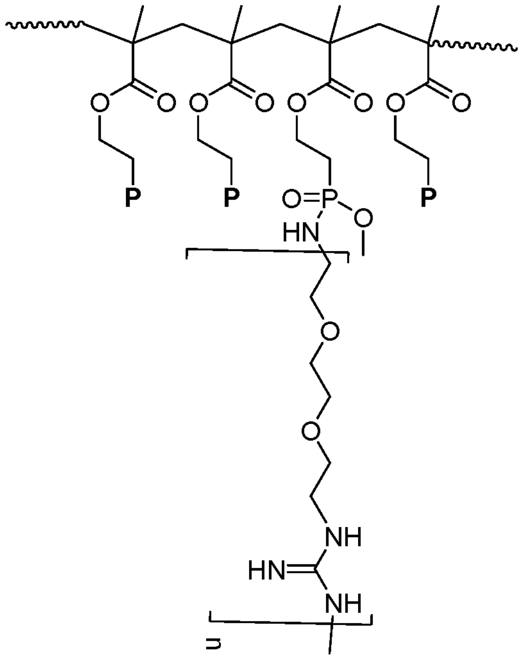

wobei P jeweils eine Phosphonatgruppe darstellt, die nicht hydrolysiert, teil- oder vollhydrolysiert sein kann, und wobei n eine ganze Zahl von 2 bis 10, vorzugsweise von 3 bis 6 darstellt, und insbesondere 5 ist. Eine Welle in dem (Meth)Acrylsäure basierten Rückgrat stellt eine Bindungsposition zu weiteren (Meth)Acrylsäure-Einheiten dar. Enthält das Pfropfcopolymer die vorstehende Struktureinheit, werden eine besonders gute antibakterielle Wirkung, gute Haftung am Implantat und gleichzeitig sehr gute Zellkompatibilität erhalten.

Die vorstehend genannten Eigenschaften können noch weiter verbessert werden, wenn, wie gemäß einer weiteren Ausführungsform offenbart, das Pfropfcopolymer mindestens eine der nachfolgenden Struktureinheiten aufweist:

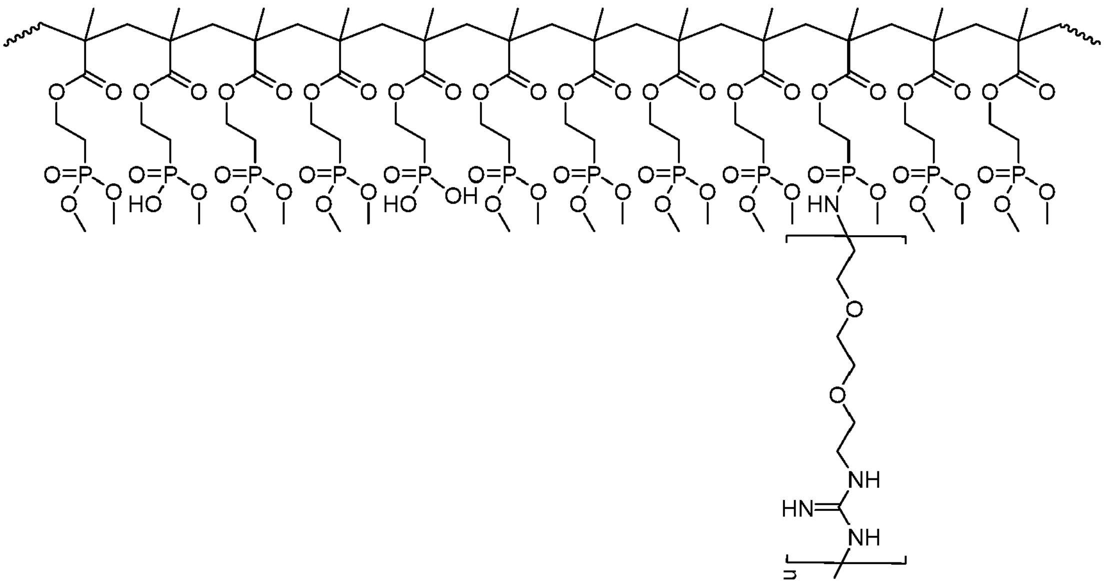

Struktureinheit A

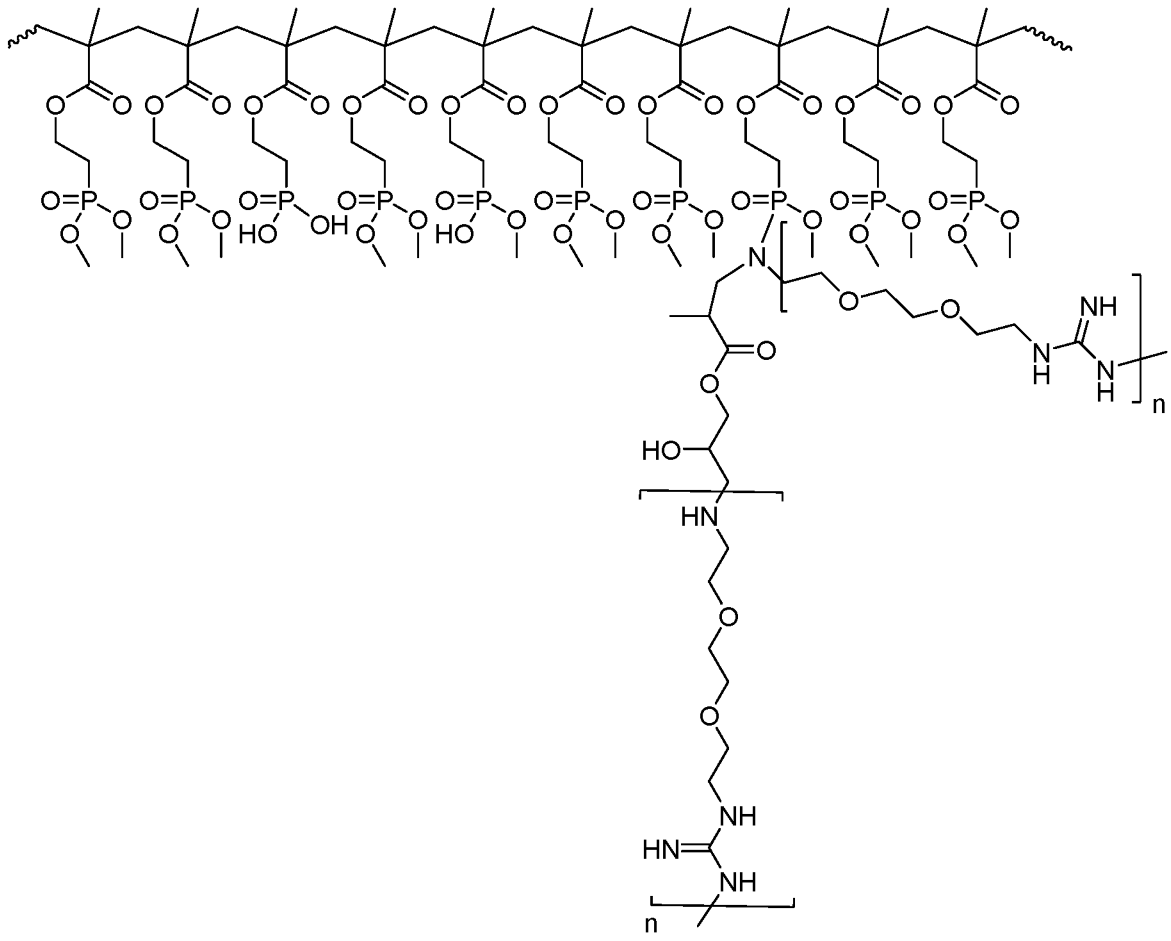

Struktureinheit B.

In den angegebenen Struktureinheiten ist n jeweils eine ganze Zahl von 2 bis 10, vorzugsweise von 3 bis 6, und ist insbesondere 5. Eine Welle in dem (Meth)Acrylsäure basierten Rückgrat stellt eine Bindungsposition zu weiteren (Meth)Acrylsäure-Einheiten dar.

Vorzugsweise enthält die antibakterielle Implantatbeschichtungszusammensetzung lediglich das vorstehend offenbarte Pfropfcopolymer und ggf. nicht oder nicht vollständig umgesetzte Edukte aus der Herstellung des Pfropfcopolymers. Dieses stellt die funktionelle Komponente der antibakteriellen Implantatbeschichtungszusammensetzung dar. Die Implantatbeschichtungszusammensetzung ist dadurch sehr gut haltbar und lagerbar, ohne dass sie einen Wirksamkeitsverlust erleidet. Um die antibakterielle Implantatbeschichtungszusammensetzung auf ein Implantat zu applizieren, kann sie jedoch ferner ein Lösungsmittel oder Dispergiermittel, wie z.B. Wasser, Ethanol, Methanol oder auch beliebige Mischungen dieser Substanzen enthalten. In den vorstehend genannten Lösungsmitteln ist das Pfropfcopolymer sehr gut lösbar oder zumindest dispergierbar und daher einfach zu verarbeiten und zu applizieren.

Die antibakterielle Implantatbeschichtungszusammensetzung kann darüber hinaus, falls erforderlich, weitere Komponenten enthalten, die jedoch die Wirksamkeit des Pfropfcopolymers nicht beeinträchtigen.

Ebenfalls erfindungsgemäß wird auch ein Verfahren zum Beschichten eines Implantats mit einer antibakteriellen Beschichtung offenbart. Hierzu wird in einem Verfahrensschritt die vorstehend beschriebene Implantatbeschichtungszusammensetzung in einem Lösungsmittel gelöst oder dispergiert. Sofern eine Lösung der Implantatbeschichtungszusammensetzung erhalten wird, so kann diese auch kolloidal sein. Als Lösungsmittel kann z.B. Wasser, Ethanol oder Methanol oder auch beliebige Mischungen dieser Substanzen eingesetzt werden. Sodann wird ein Implantat bereitgestellt, das für die Weiterbearbeitung entfettet sein sollte. Das Entfetten kann z.B. durch Reinigung mit Lösungsmitteln wie Aceton, Dichlormethan, Methanol und dergleichen oder mittels Plasmareinigung erfolgen. Ggf. nach Trocknung erfolgt anschließend das Aufbringen der gelösten oder dispergierten Implantatbeschichtungszusammensetzung auf das Implantat. Dadurch, dass die Implantatbeschichtungszusammensetzung in Form einer Lösung oder Dispersion vorliegt, können viele einfache, herkömmliche Applikationsprozesse Anwendung finden, was den technischen Aufwand für das Verfahren geringhält. Auch wird so eine Applikation auf geometrisch komplexen Oberflächen vereinfacht. Abschließend erfolgt ein Binden der Implantatbeschichtungszusammensetzung an das Implantat. Dies wird durch Einwirken von Temperaturen zwischen 50 und 200 °C z.B. in einem Trockenschrank oder in einem geeigneten Ofen ausgeführt. Durch den Bindeschritt zieht die Implantatbeschichtungszusammensetzung auf die Oberfläche des Implantats auf und bindet durch die Phosphonatgruppen kovalent an das Implantat. Die Bindungskraft der Bindung zwischen der Implantatoberfläche und der Implantatbeschichtungszusammensetzung ist aufgrund des hohen Anteils an Phosphonat-Seitenketten hoch. Das Aufbringen eines Adhäsivs kann somit entfallen. Durch das Verfahren wird ohne hohen technischen Aufwand ein Implantat mit dauerhaft hoher antimikrobieller Wirksamkeit bei sehr guter Zellkompatibilität erhalten.

Die vorstehend für die antimikrobielle Implantatbeschichtungszusammensetzung dargelegten Vorteile, vorteilhaften Weiterbildungen und Ausgestaltungen finden auch Anwendung auf das erfindungsgemäße Verfahren zum Beschichten eines Implantats mit einer antibakteriellen Beschichtung.

Zur Aufreinigung der Beschichtung können sich nach dem Binden der Implantatbeschichtungszusammensetzung ein Waschschritt und ein Trocknungsschritt anschließen.

Des Weiteren kann das Implantat vor dem Beschichten übliche Vorbereitungsschritte durchlaufen, wie z.B. ein Polieren der Oberfläche, um eine gewünschte Oberflächenrauheit, beispielsweise eine Oberflächenrauheit von 0,013 μm oder darunter, zu erhalten.

Eine weitere vorteilhafte Weiterbildung des Verfahrens sieht vor, dass das Aufbringen der Lösung der Implantatbeschichtungszusammensetzung durch Spin Coating (insbesondere bei planaren Substraten), ein Tauch- oder Sprühverfahren ausgeführt wird. Hierdurch wird die Verfahrensführung vereinfacht und eine Beschichtung mit besonders gleichmäßiger Schichtdicke erhalten.

Eine sehr gute Reaktionsfähigkeit der Implantatbeschichtungszusammensetzung mit der Implantatoberfläche unter Ausbildung einer ebenmäßigen Schichtdicke wird vorteilhaft dadurch erhalten, dass eine Konzentration der Implantatbeschichtungszusammensetzung im Lösungsmittel 2 bis 20 mg/ml und insbesondere 8 bis 12 mg/ml beträgt.

Des Weiteren erfindungsgemäß wird auch ein antimikrobiell beschichtetes Implantat beschrieben. Das antimikrobiell beschichtete Implantat umfasst eine aus der vorstehend beschriebenen Implantatbeschichtungszusammensetzung gebildete Beschichtung. Die Beschichtung kann dabei nach dem vorstehend beschriebenen Verfahren zum Beschichten eines Implantats mit einer antibakteriellen Beschichtung hergestellt sein. Das erfindungsgemäße Implantat ist aus Titan, Zirkon, Tantal, Edelstahl, Zirkonoxid oder PEEK gebildet und weist eine durchschnittliche Schichtdicke der antimikrobiellen Beschichtung von 5 bis 50 nm und insbesondere von 15 bis 25 nm auf. Die Schichtdicke wird dabei durch Ellipsometrie bestimmt. Die durch das erfindungsgemäße Verfahren im erfindungsgemäßen Implantat erhaltene Schichtdicke liegt um etwa 10 nm höher als diejenige, die durch alternative Herstellverfahren einer Beschichtungszusammensetzung unter sonst gleichen Bedingungen erhalten werden. Dies deutet darauf hin, dass die chemische Struktur der Implantatbeschichtungszusammensetzung nicht mit Strukturen herkömmlicher antibakterieller Beschichtungen vergleichbar ist. Beispielhafte erfindungsgemäße Implantate umfassen dentale Implantate, Implantate für Hüft- und Knieendoprothesen sowie Herzschrittmacher. Insbesondere in der Tumortherapie, wenn immunsupprimierte Patienten große Implantate erhalten, ist eine Infektionsprophylaxe von enormer Bedeutung. Insbesondere für dentale Implantate, die naturgemäß in Kontakt mit der bakteriell stark belasteten Mundhöhle des Patienten stehen und deshalb einem besonderen Infektionsrisiko ausgesetzt sind, ist die vorliegende Erfindung besonders geeignet.

Beispiel

Nachstehend wird die Synthese eines in der erfindungsgemäßen antibakteriellen Implantatbeschichtungszusammensetzung verwendeten Pfropfcopolymers beschrieben:
Die Synthese wurde in zwei Stufen durchgeführt. In der ersten Stufe wurden 2,2'-(Ethylendioxy)diethylamin und Guanidinhydrochlorid bei 170 °C unter Verwendung eines mechanischen Rührers zu dem oligomeren Produkt Poly-(2-(2-ethoxy)-ethoxyethyl-guanidinhydrochlorid) (PEDBEG) kondensiert. Das oligomere Guanidin umfasste primäre Amine, die als funktionelle Gruppe Reaktionen eingehen und, wie im nächsten Schritt dargelegt, kovalente N-P-Bindungen mit Phosphonatgruppen eingehen können.

In der zweiten Stufe wurde 2-(Dimethoxyphosphoryl)ethyl methacrylat (DMMEP) in Gegenwart des in der ersten Stufe erhaltenen PEDBEG-Oligomers für 14 h bei 60 °C polymerisiert. Als Initiator diente 2,2'-Azobis(2-methylpropionitril). In dieser Reaktion erfolgte der Aufbau des Poly(DMMEP)-Rückgrats und die Reaktion der Phosphonat-Seitenketten mit dem PEDBEG. Das Produkt dieser Reaktion stellte neben reinem PEDBEG und Poly(2-(dimethoxyphosphoryl)ethylmethacrylat) ein Propfcopolymer dar, das über eine Phosphor-Stickstoff-Bindung gebundenes PEDBEG als Seitengruppe beinhaltete. Des Weiteren waren die Phosphonatgruppen zu etwa 7-20 Mol% hemi- bzw. vollhydrolysiert.

Die nachfolgende Tabelle gibt eine prozentuale Zusammensetzung der Phosphoratome in den entsprechenden Gruppen für verschiedenen Chargen der Pfropfcopolymerherstellung an:

| | Phosphonat | Phosphonamidat | Phosphonsäure | Hemi Phosphonsäure |
|---|---|---|---|---|
| Charge AS1 | 78,66% | 2,07% | 13,01% | 2,89% |

(fortgesetzt)

| | Phosphonat | Phosphonamidat | Phosphonsäure | Hemi Phosphonsäure |
|---|---|---|---|---|
| Charge AS2 | 78,07% | 1,77% | 14,01% | 3,04% |
| Charge AS3 | 75,37% | 2,50% | 13,08% | 5,16% |
| Charge LF1 | 82,94% | 3,20% | 12,24% | 1,12% |
| Gemittelt | 79% | 2,4% | 13% | 3,0% |
| Von - bis | 75 - 83% | 1,8 - 3,2% | 12 - 14% | 1 - 5% |
| Massenanteil* | 78 | 8% | 11% | 3% |
| * Der entsprechenden Strukturen, berechnet mit dem Mittelwert und unter Annahme eines mittleren Molekulargewichts von 537 g/mol für die verwendeten PEDBEG-Oligomere. | | | | |

Weitere Einzelheiten, Vorteile und Merkmale der vorliegenden Erfindung ergeben sich aus nachfolgender Beschreibung von Ausführungsbeispielen anhand der Zeichnung. Es zeigt:

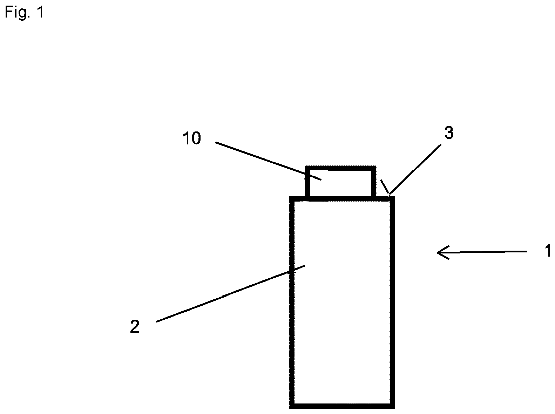

Fig. 1 eine schematische Darstellung eines mit einer antibakteriellen Beschichtung beschichteten Implantats gemäß einer Ausführungsform der Erfindung.

Fig. 1 zeigt im Detail eine schematische Darstellung eines mit einer antibakteriellen Beschichtung 10 beschichteten Implantats 1 gemäß einer Ausführungsform der Erfindung. Das antibakteriell beschichtete Implantat 1 umfasst einen Grundkörper 2, der beispielsweise Titan, Zirkon, Tantal, Edelstahl, Zirkonoxid oder PEEK umfasst. Auf eine Oberfläche 3 des Grundkörpers 2 ist die antibakterielle Beschichtung 10 mit einer durchschnittlichen Schichtdicke S von 5 bis 50 nm und insbesondere 15 bis 25 nm angeordnet.

Die antibakterielle Beschichtung 10 ist aus einer antibakteriellen Implantatbeschichtungszusammensetzung gebildet, die ein Pfropfcopolymer mit einem (Meth)Acrylsäure basierten Rückgrat und Phosphonat-Seitenketten umfasst, wobei mindestens eine Phosphonat-Seitenkette mit einem Guanidin-Oligomer durch eine N-P-Bindung verbunden ist. Mindestens eine weitere Phosphonat-Seitenkette, die nicht durch eine N-P-Bindung mit einem Guanidin-Oligomer verbunden ist, ist an die Implantatoberfläche 3 gebunden und sorgt somit für eine gute Haftung des Pfropfcopolymers am Grundkörper 2.

Die antibakterielle Beschichtung 10 auf dem Grundkörper 2 des Implantats wurde wie folgt erhalten:
Zunächst wurde die vorstehend beschriebene Implantatbeschichtungszusammensetzung in einem Lösungsmittel, wie z.B. Wasser, Ethanol oder Methanol, gelöst oder dispergiert. Sodann wurde ein zu beschichtendes Implantat bereitgestellt und entfettet. Anschließend wurde die Lösung bzw. Dispersion der Implantatbeschichtungszusammensetzung auf die Oberfläche 3 des Grundkörpers 2 des Implantats z.B. durch Spin Coating oder ein Tauch- oder Sprühverfahren, aufgebracht und anschließend die Implantatbeschichtungszusammensetzung an das Implantat unter Einwirkung von Temperaturen in einem Bereich von 50 bis 200 °C gebunden, wodurch zwischen der Oberfläche 3 des Implantats 10 und der Implantatbeschichtungszusammensetzung durch Ausbilden von Bindungen zwischen der Oberfläche 3 des Implantats und den Phosphonat-Seitenketten des Pfropfcopolymers der Implantatbeschichtungszusammensetzung die antibakterielle Beschichtung 10 ausgebildet wurde. Hierbei betrugt eine Konzentration der Implantatbeschichtungszusammensetzung im Lösungsmittel 2 bis 20 mg/ml.

Das antibakteriell beschichtete Implantat 1 zeichnete sich aufgrund der antibakteriellen Beschichtung 10 bei hoher Zellkompatibilität durch eine dauerhaft sehr gute antibakterielle Funktionalität aus. Das Beschichten des Implantats mit der antibakteriellen Beschichtung 10 erfolgte gleichmäßig abdeckend und unter Anwendung technisch herkömmlicher Mittel.

Neben der vorstehenden schriftlichen Beschreibung der Erfindung wird zu deren ergänzender Offenbarung hiermit explizit auf die zeichnerische Darstellung der Erfindung in Fig. 1 Bezug genommen.

**Bezugszeichenliste**

1 antibakteriell beschichtetes Implantat
2 Grundkörper
3 Oberfläche

10 antibakterielle Beschichtung

**Patentansprüche**

1. Antibakterielle Implantatbeschichtungszusammensetzung umfassend ein Pfropfcopolymer umfassend:

   - ein (Meth)Acrylsäure basiertes Rückgrat und
   - Phosphonat-Seitenketten,

   wobei mindestens eine Phosphonat-Seitenkette mit einem Guanidin-Oligomer durch eine N-P-Bindung verbunden ist.

2. Antibakterielle Implantatbeschichtungszusammensetzung nach Anspruch 1, wobei ein Anteil an Phosphonat-Seitenketten, die mit einem Guanidin-Oligomer durch eine N-P-Bindung verbunden sind, bezogen auf den Gesamtanteil an Phosphonat-Seitengruppen in Mol%, 0,8 bis 6 Mol% und insbesondere 0,9 bis 5 Mol% beträgt.

3. Antibakterielle Implantatbeschichtungszusammensetzung nach Anspruch 1 oder 2, wobei die nicht mit dem Guanidin-Oligomer verbundenen Phosphonat-Seitenketten nicht hydrolysiert, teilhydrolysiert oder vollhydrolysiert sind.

4. Antibakterielle Implantatbeschichtungszusammensetzung nach Anspruch 3, wobei ein Anteil an teilhydrolysierten und vollhydrolysierten Phosphonat-Seitengruppen, bezogen auf den Gesamtanteil an Phosphonat-Seitengruppen in Mol%, 5 bis 30 Mol% und insbesondere 7 bis 20 Mol% beträgt.

5. Antibakterielle Implantatbeschichtungszusammensetzung nach einem der vorhergehenden Ansprüche, wobei das Molekulargewicht des Pfropfcopolymers (Mw) nachfolgende Gleichung erfüllt:

$$5kDa < Mw < 40kDa.$$

6. Antibakterielle Implantatbeschichtungszusammensetzung nach einem der vorhergehenden Ansprüche, wobei das Guanidin-Oligomer mindestens eine durch die nachfolgenden Formeln dargestellte Verbindung ist:

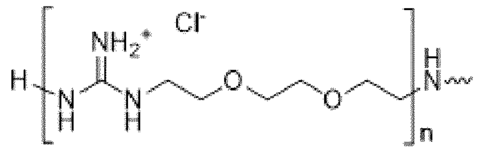

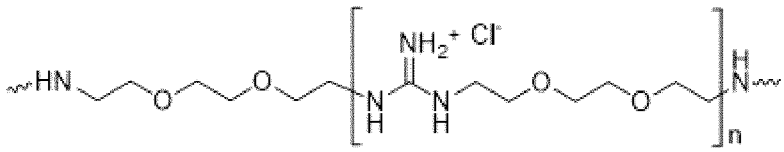

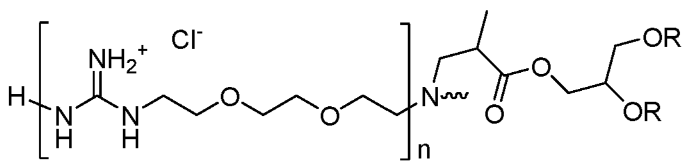

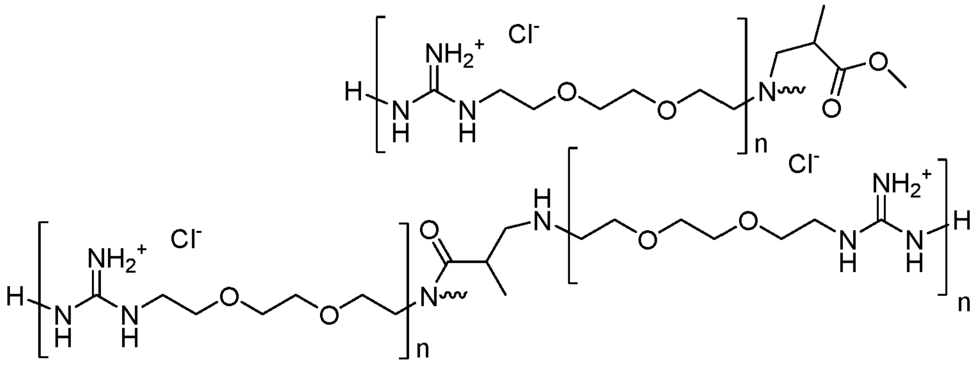

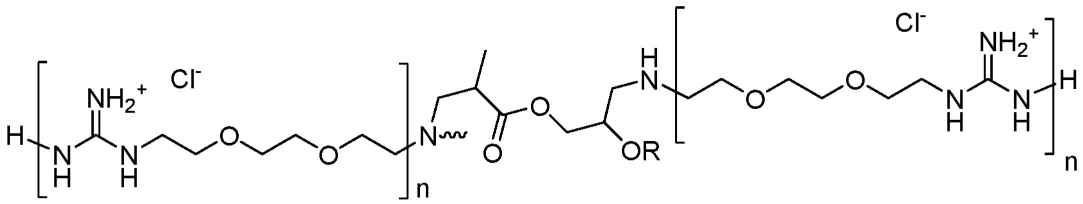

wobei n jeweils eine ganze Zahl von 2 bis 10, vorzugsweise von 3 bis 6, und insbesondere 5 ist und wobei "~" eine Bindungsposition an das Phosphoratom einer Phosphonat-Seitenkette darstellt.

7. Antibakterielle Implantatbeschichtungszusammensetzung nach einem der vorhergehenden Ansprüche, wobei das Molekulargewicht des Guanidin-Oligomers 800 bis 1300 g/mol beträgt.

8. Antibakterielle Implantatbeschichtungszusammensetzung nach einem der vorhergehenden Ansprüche, wobei das Pfropfcopolymer nachfolgende Struktureinheit aufweist:

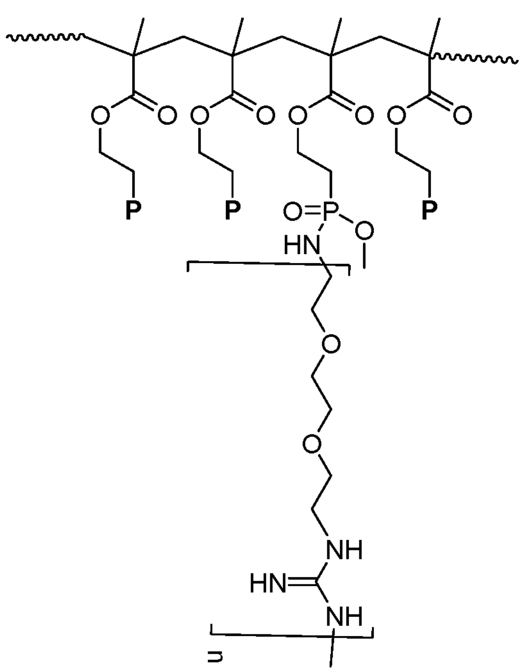

wobei P jeweils eine Phosphonatgruppe darstellt, wobei n eine ganze Zahl von 2 bis 10, vorzugsweise von 3 bis 6 darstellt, und insbesondere 5 ist und wobei eine Welle im (Meth)Acrylsäure basierten Rückgrat eine Bindungsposition an eine benachbarte (Meth)Acrylsäure Einheit darstellt.

9. Antibakterielle Implantatbeschichtungszusammensetzung nach einem der vorhergehenden Ansprüche, wobei das

Pfropfcopolymer mindestens eine der nachfolgenden Struktureinheiten aufweist:

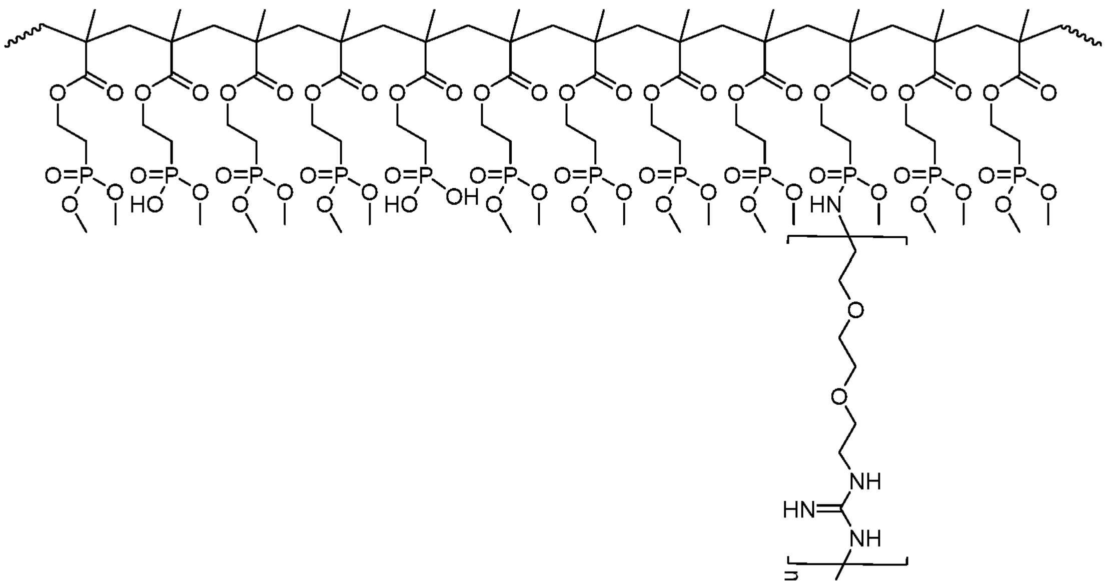

Struktureinheit A

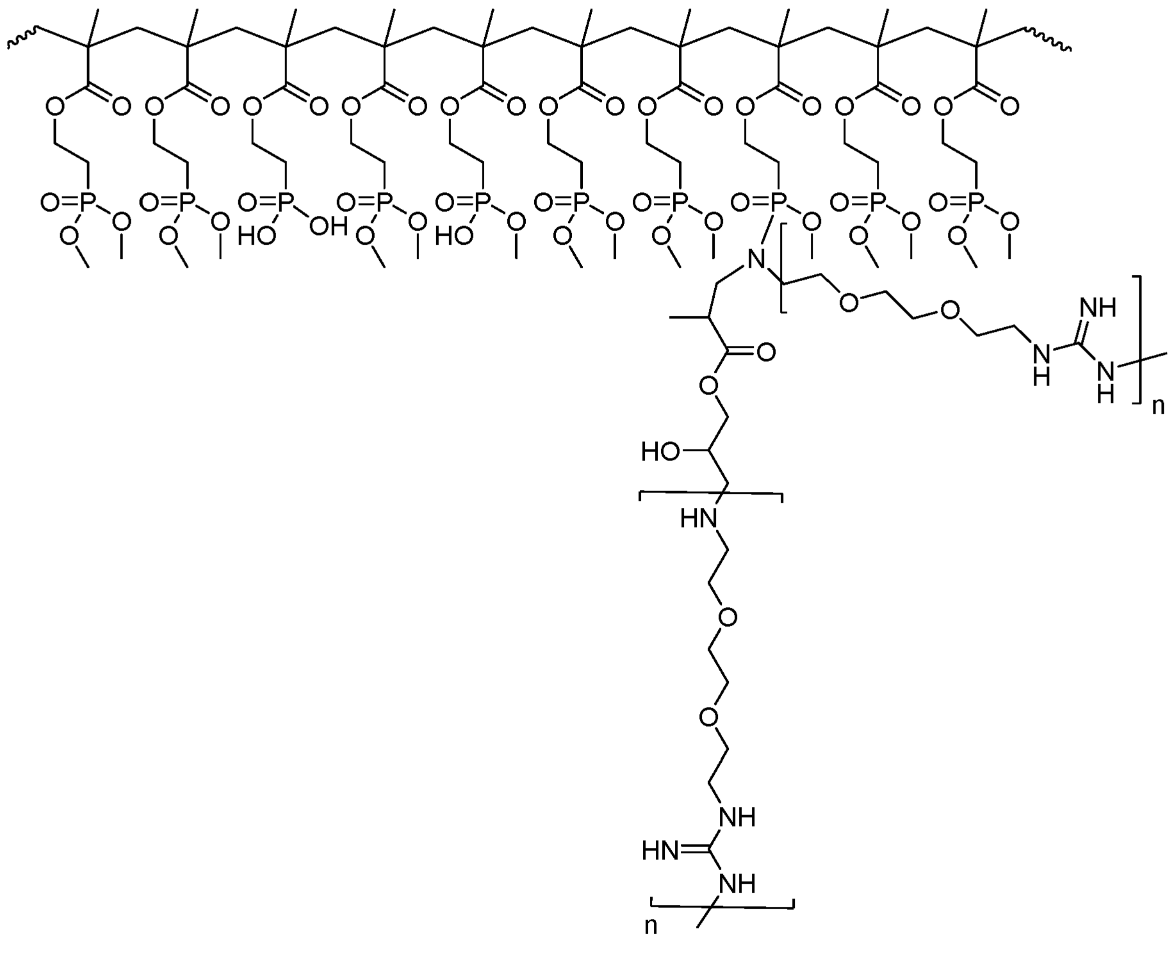

Struktureinheit B

wobei n jeweils eine ganze Zahl von 2 bis 10, vorzugsweise von 3 bis 6 darstellt, und insbesondere 5 ist, und wobei eine Welle im (Meth)Acrylsäure basierten Rückgrat eine Bindungsposition an eine benachbarte (Meth)Acrylsäure

Einheit darstellt.

**10.** Antibakterielle Implantatbeschichtungszusammensetzung nach einem der vorhergehenden Ansprüche, ferner umfassend Wasser.

**11.** Verfahren zum Beschichten eines Implantats mit einer antibakteriellen Beschichtung umfassend die Schritte:

• Lösen oder Dispergieren der Implantatbeschichtungszusammensetzung nach einem der vorhergehenden Ansprüche in einem Lösungsmittel,
• Bereitstellen und Entfetten eines Implantats,
• Aufbringen der Lösung bzw. Dispersion der Implantatbeschichtungszusammensetzung auf das Implantat und
• Binden der Implantatbeschichtungszusammensetzung an das Implantat unter Einwirkung von Temperaturen in einem Bereich von 50 bis 200 °C.

**12.** Verfahren nach Anspruch 11, ferner umfassend einen Waschschritt und einen anschließenden Trocknungsschritt nach dem Binden der Implantatbeschichtungszusammensetzung.

**13.** Verfahren nach Anspruch 11 oder 12, **dadurch gekennzeichnet, dass** das Aufbringen der Lösung bzw. der Dispersion der Implantatbeschichtungszusammensetzung durch Spin Coating oder ein Tauch- oder Sprühverfahren ausgeführt wird.

**14.** Verfahren nach einem der Ansprüche 11 bis 13, **dadurch gekennzeichnet, dass** eine Konzentration der Implantatbeschichtungszusammensetzung im Lösungsmittel 2 bis 20 mg/ml und insbesondere 8 bis 12 mg/ml, beträgt.

**15.** Antibakteriell beschichtetes Implantat umfassend eine aus der Implantatbeschichtungszusammensetzung nach einem der Ansprüche 1 bis 10 gebildete Beschichtung.

**16.** Antibakteriell beschichtetes Implantat nach Anspruch 15, wobei das Implantat Titan, Zirkon, Tantal, Edelstahl, Zirkonoxid oder PEEK umfasst und/oder wobei eine durchschnittliche Schichtdicke der antibakteriellen Beschichtung 5 bis 50 nm und insbesondere 15 bis 25 nm beträgt.

## Claims

**1.** Antibacterial implant coating composition comprising a graft copolymer, comprising:

- a (meth)acrylic acid-based backbone and
- phosphonate side chains,

wherein at least one phosphonate side chain is linked to a guanidine oligomer by an N-P bond.

**2.** Antibacterial implant coating composition according to claim 1, wherein, based on the total proportion of phosphonate side groups in mol.%, a proportion of phosphonate side chains which are linked to a guanidine oligomer by an N-P bond is 0.8 to 6 mol.% and in particular 0.9 to 5 mol.%.

**3.** Antibacterial implant coating composition according to claim 1 or 2, wherein the phosphonate side chains that are not linked to the guanidine oligomer are not hydrolysed, partially hydrolysed or fully hydrolysed.

**4.** Antibacterial implant coating composition according to claim 3, wherein a proportion of partially hydrolysed and fully hydrolysed phosphonate side groups, based on the total proportion of phosphonate side groups in mol.%, is 5 to 30 mol.% and in particular 7 to 20 mol.%.

**5.** Antibacterial implant coating composition according to any of the preceding claims, wherein the molecular weight of the graft copolymer (Mw) satisfies the following equation:

$$5\ \text{kDa} < M_w < 40\ \text{kDa}.$$

6. Antibacterial implant coating composition according to any of the preceding claims, wherein the guanidine oligomer is at least one compound represented by the following formulas:

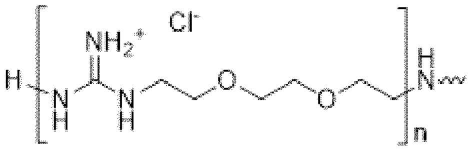

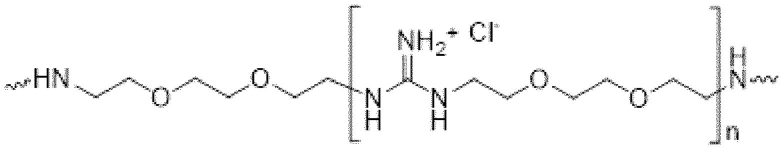

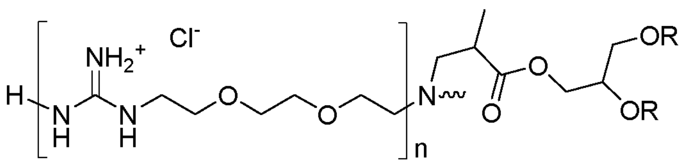

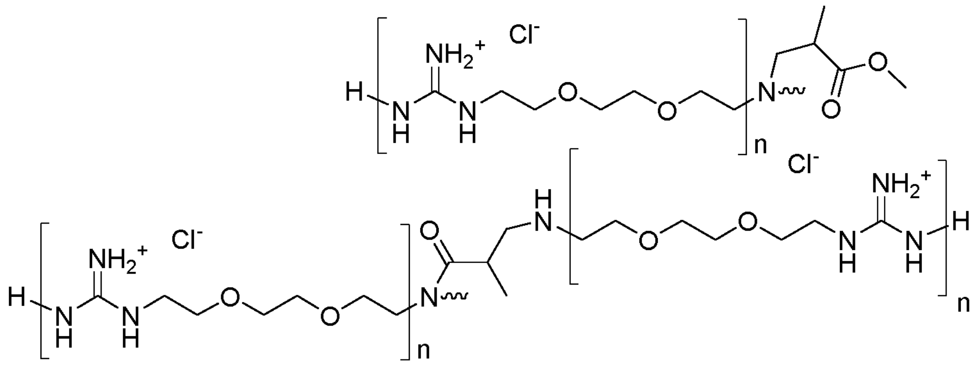

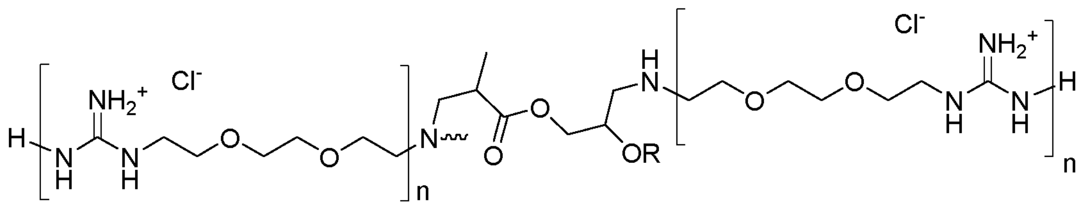

wherein n is in each case an integer from 2 to 10, preferably from 3 to 6, and in particular 5, and wherein "-" represents a binding position to the phosphor atom of a phosphonate side chain.

7. Antibacterial implant coating composition according to any of the preceding claims, wherein the molecular weight of the guanidine oligomer is 800 to 1300 g/mol.

8. Antibacterial implant coating composition according to any of the preceding claims, wherein the graft copolymer has the following structural unit:

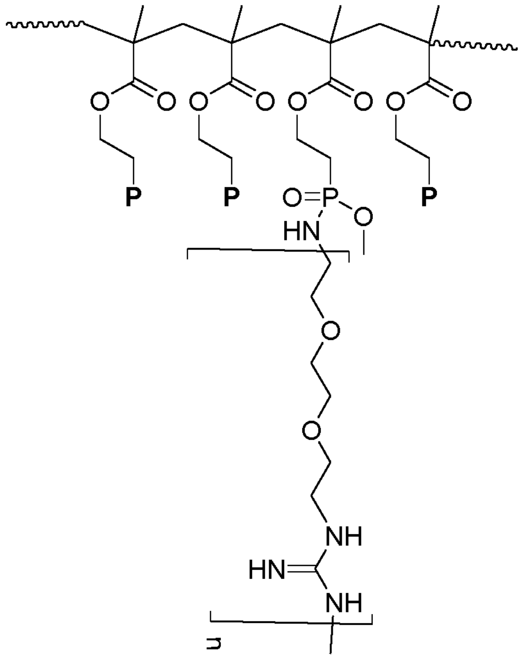

wherein P in each case represents a phosphonate group, wherein n represents an integer from 2 to 10, preferably from 3 to 6, and in particular is 5, and wherein a wave in the (meth)acrylic acid-based backbone represents a binding position to an adjacent (meth)acrylic acid unit.

9. Antibacterial implant coating composition according to any of the preceding claims, wherein the graft copolymer has at least one of the following structural units:

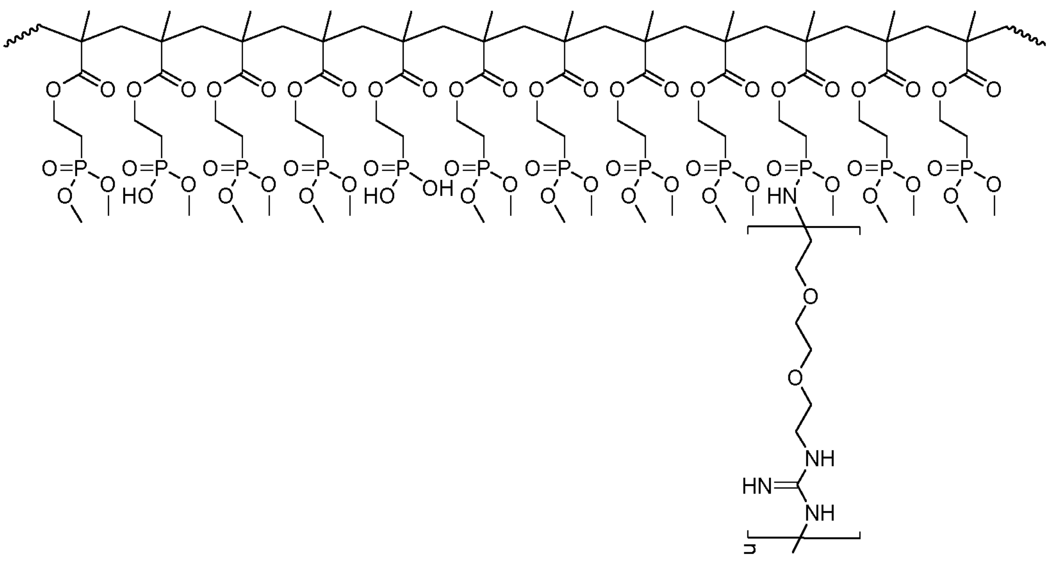

Structural unit A

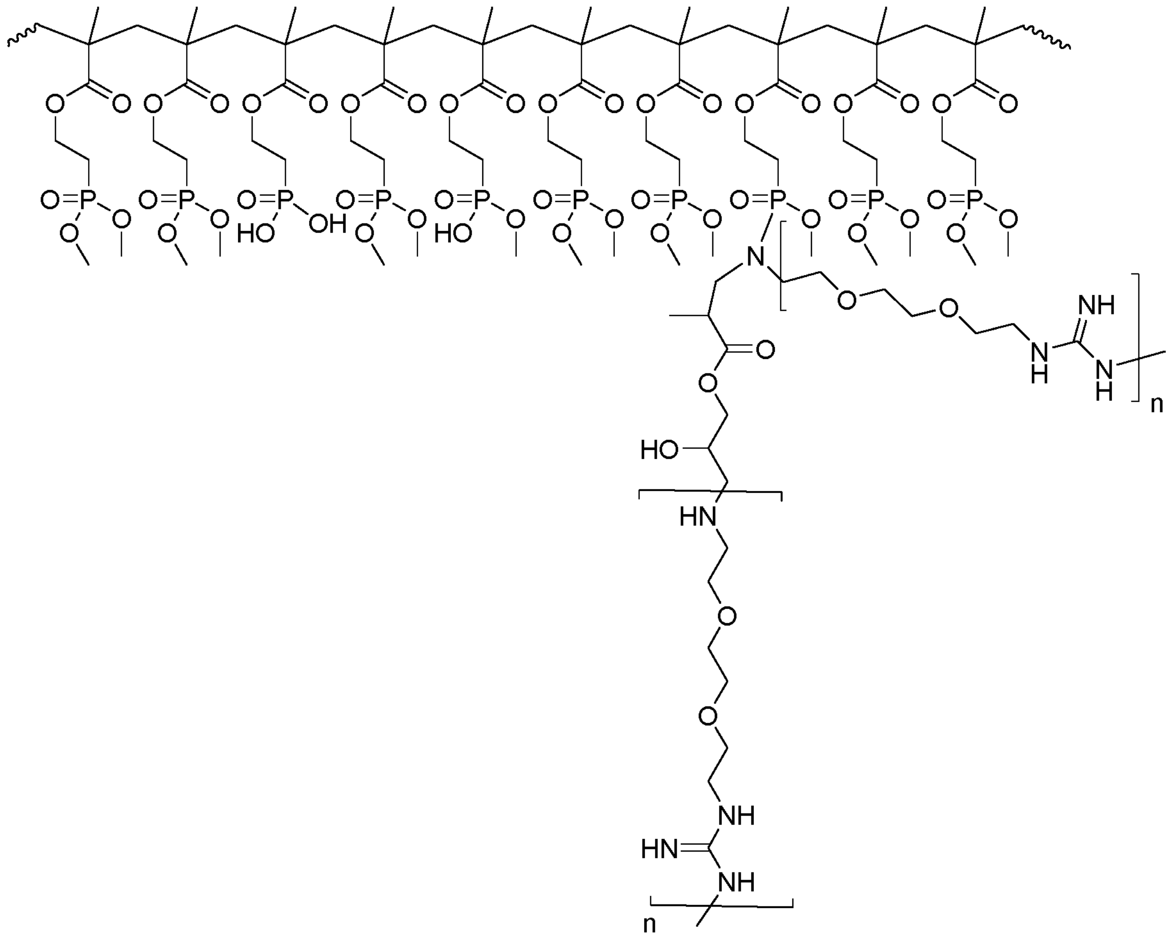

Structural unit B

wherein n in each case represents an integer from 2 to 10, preferably from 3 to 6, and in particular is 5, and wherein a wave in the (meth)acrylic acid-based backbone represents a binding position to an adjacent (meth)acrylic acid unit.

**10.** Antibacterial implant coating composition according to any of the preceding claims, further comprising water.

**11.** Method for coating an implant using an antibacterial coating, comprising the steps of:

- dissolving or dispersing the implant coating composition according to any of the preceding claims in a solvent,
- providing and degreasing an implant,
- applying the solution or dispersion of the implant coating composition to the implant, and
- binding the implant coating composition to the implant under action of temperatures in a range from 50 to 200 °C.

**12.** Method according to claim 11, further comprising a washing step and a subsequent drying step after the binding of the implant coating composition.

**13.** Method according to claim 11 or 12, **characterised in that** the application of the solution or the dispersion of the implant coating composition is carried out by spin coating or a dipping or spraying process.

**14.** Method according to any of claims 11 to 13, **characterised in that** a concentration of the implant coating composition in the solvent is 2 to 20 mg/ml and in particular 8 to 12 mg/ml.

**15.** Antibacterially coated implant, comprising a coating formed from the implant coating composition according to any of claims 1 to 10.

**16.** Antibacterially coated implant according to claim 15, wherein the implant comprises titanium, zirconium, tantalum, stainless steel, zirconium oxide or PEEK, and/or wherein an average layer thickness of the antibacterial coating is

5 to 50 nm and in particular 15 to 25 nm.

## Revendications

**1.** Composition antibactérienne de revêtement d'implant comprenant un copolymère greffé comprenant :

- un squelette à base d'acide (méth)acrylique, et
- des chaînes latérales de phosphonate,

dans laquelle au moins une chaîne latérale de phosphonate est liée par une liaison N-P à un oligomère de guanidine.

**2.** Composition antibactérienne de revêtement d'implant selon la revendication 1, dans laquelle une fraction de chaînes latérales de phosphonate, qui sont reliées à un oligomère de guanidine par une liaison N-P, va de 0,8 à 6 % mol. et en particulier de 0,9 à 5 % mol. par rapport à la fraction totale de groupes latéraux de phosphonate.

**3.** Composition antibactérienne de revêtement d'implant selon la revendication 1 ou 2, dans laquelle les chaînes latérales de phosphonate non reliées à l'oligomère de guanidine ne sont pas hydrolysées, sont partiellement hydrolysées ou sont totalement hydrolysées.

**4.** Composition antibactérienne de revêtement d'implant selon la revendication 3, dans laquelle une fraction de groupes latéraux de phosphonate partiellement hydrolysés et totalement hydrolysés va de 5 à 30 % mol. et en particulier de 7 à 20 % mol. par rapport à la fraction totale de groupes latéraux de phosphonate.

**5.** Composition antibactérienne de revêtement d'implant selon l'une quelconque des revendications précédentes, dans laquelle le poids moléculaire du copolymère greffé (Mw) remplit l'équation suivante :

$$5kDa < Mw < 40kDa.$$

**6.** Composition antibactérienne de revêtement d'implant selon l'une quelconque des revendications précédentes, dans laquelle l'oligomère de guanidine est au moins un composé représenté par les formules suivantes :

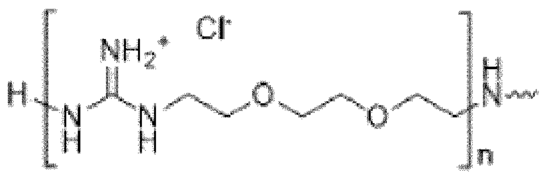

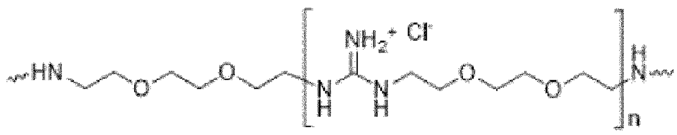

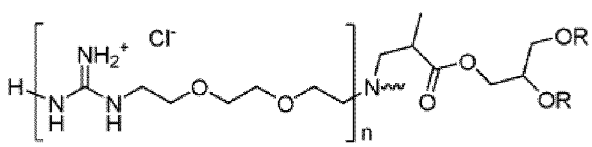

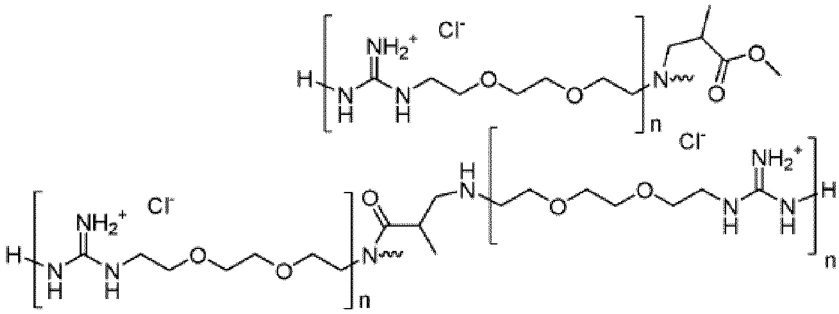

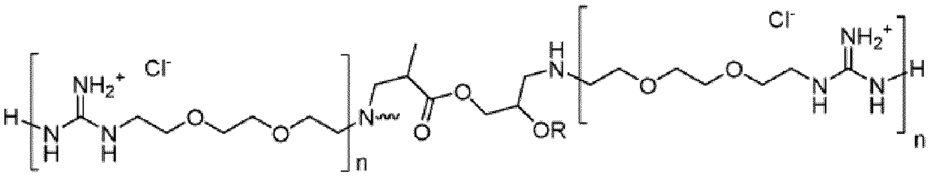

dans laquelle n représente respectivement un nombre entier de 2 à 10, de préférence de 3 à 6, et en particulier est 5 et dans laquelle « ~ » représente une position de liaison à l'atome de phosphore d'une chaîne latérale de phosphonate.

7. Composition antibactérienne de revêtement d'implant selon l'une quelconque des revendications précédentes, dans laquelle le poids moléculaire de l'oligomère de guanidine va de 800 à 1300 g/mole.

8. Composition antibactérienne de revêtement d'implant selon l'une quelconque des revendications précédentes, dans laquelle le copolymère greffé présente un motif structural suivant :

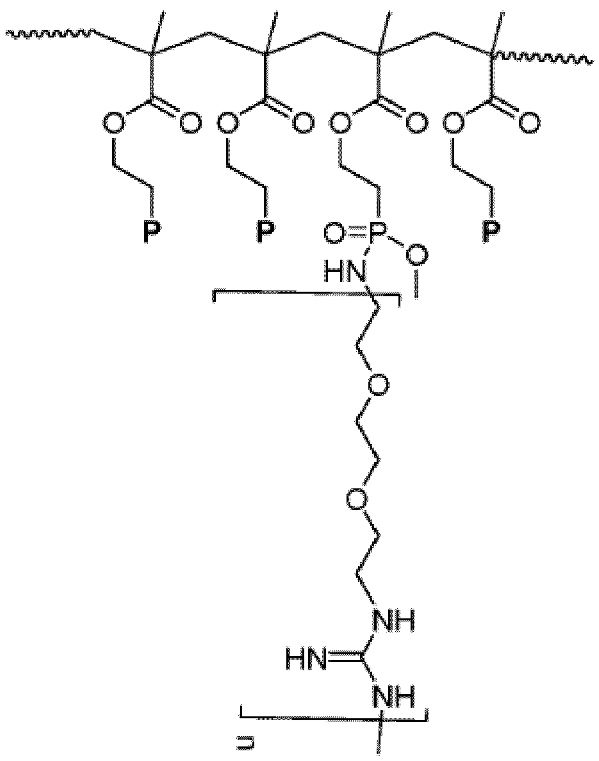

dans laquelle P représente respectivement un groupe phosphonate, dans laquelle n représente un nombre entier de 2 à 10, de préférence de 3 à 6 et est en particulier 5 et dans laquelle une ondulation dans le squelette à base d'acide (méth)acrylique représente une position de liaison sur un motif d'acide (méth)acrylique adjacent.

**9.** Composition antibactérienne de revêtement d'implant selon l'une quelconque des revendications précédentes, dans laquelle le copolymère greffé présente au moins un des motifs structuraux suivants :

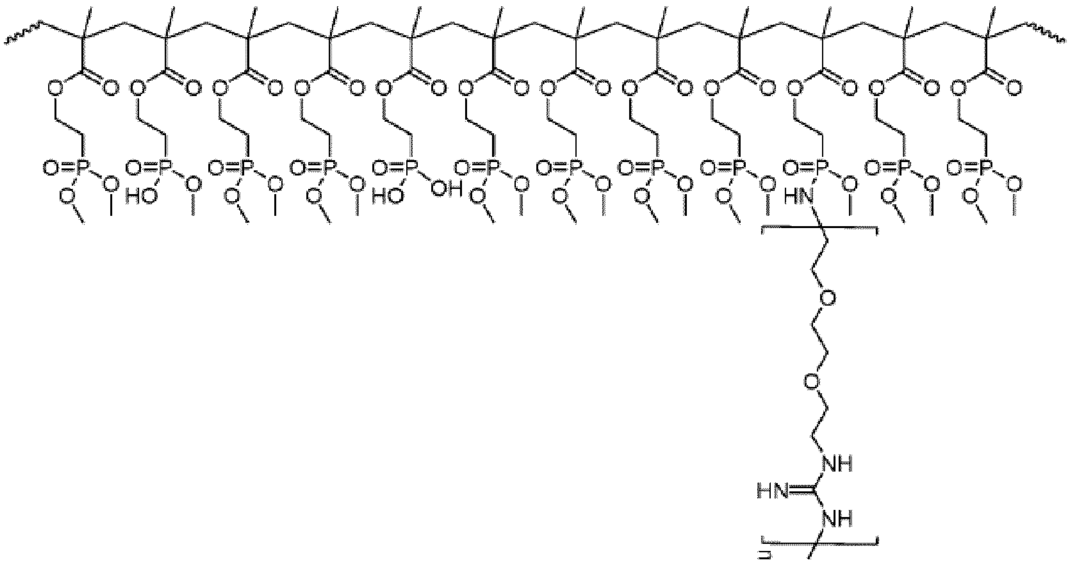

Motif structural A

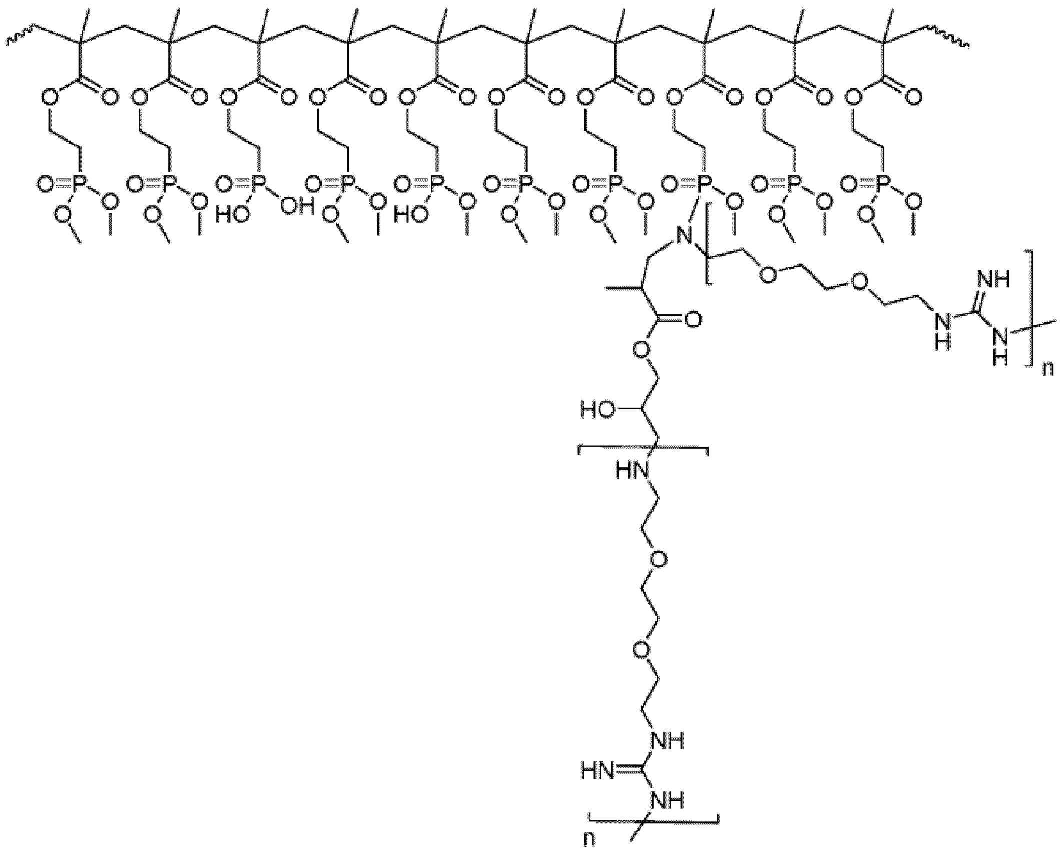

Motif structural B dans laquelle n représente respectivement un nombre entier de 2 à 10, de préférence de 3 à 6, et est en particulier 5, et dans laquelle une ondulation dans le squelette à base d'acide (méth)acrylique représente une position de liaison sur un motif d'acide (méth)acrylique adjacent.

**10.** Composition antibactérienne de revêtement d'implant selon l'une quelconque des revendications précédentes,

comprenant en outre de l'eau.

**11.** Procédé de revêtement d'un implant avec un revêtement antibactérien, comprenant les étapes :

- de dissolution ou de dispersion de la composition de revêtement d'implant selon l'une quelconque des revendications précédentes, dans un solvant,
- de fourniture et de dégraissage d'un implant,
- d'application de la solution ou de dispersion de la composition de revêtement d'implant sur l'implant, et
- de liaison de la composition de revêtement d'implant à l'implant sous l'action de températures dans une plage de 50 à 200 °C.

**12.** Procédé selon la revendication 11, comprenant en outre une étape de lavage et une étape de séchage qui suit après la liaison de la composition de revêtement d'implant.

**13.** Procédé selon la revendication 11 ou 12, **caractérisé en ce que** l'application de la solution ou de la dispersion de la composition de revêtement d'implant est exécutée par revêtement par centrifugation ou par un procédé d'immersion ou de pulvérisation.

**14.** Procédé selon l'une quelconque des revendications 11 à 13, **caractérisé en ce qu'**une concentration de la composition de revêtement d'implant dans le solvant va de 2 à 20 mg/ml et en particulier de 8 à 12 mg/ml.

**15.** Implant à revêtement antibactérien comprenant un revêtement formé à partir de la composition de revêtement antibactérienne selon l'une quelconque des revendications 1 à 10.

**16.** Implant à revêtement antibactérien selon la revendication 15, dans lequel l'implant comprend du titane, du zirconium, du tantale, de l'acier inoxydable, de l'oxyde de zirconium ou du PEEK (polyétheréthercétone) et/ou dans lequel une épaisseur de couche moyenne du revêtement antibactérien va de 5 à 50 nm et en particulier de 15 à 25 nm.

Fig. 1